# EUROPÄISCHE PATENTSCHRIFT

(11) **EP 0 841 010 B1**
(45) Veröffentlichungstag und Bekanntmachung des Hinweises auf die Patenterteilung: **03.07.2002**
(21) Anmeldenummer: 97119013.7
(22) Anmeldetag: 31.10.1997
(51) Int. Cl.: A23L 1/30, A23D 9/007

(54) **Stabile, kaltwasser-dispergierbare Präparate**
Stable compositions dispersible in cold water
Compositions stables et dispersables dans l'eau froide

(30) Priorität: 06.11.1996 CH 274196
(43) Veröffentlichungstag der Anmeldung: 13.05.1998
(73) Patentinhaber: F. HOFFMANN-LA ROCHE AG, 4070 Basel (CH)
(72) Erfinder: Tritsch, Jean-Claude, 68300 Saint-Louis (FR); Ulm, Johann, 4104 Oberwil (CH)
(74) Vertreter: Kellenberger, Marcus, Dr.

(56) Entgegenhaltungen:
- EP-A- 0 347 751
- WO-A-96/20612
- WO-A-96/26647
- DE-A- 3 603 000
- DATABASE WPI Section Ch, Week 9317 Derwent Publications Ltd., London, GB; Class D13, AN 93-140541 XP002056752 & JP 05 078 692 A (NIPPON OILS & FATS CO LTD) , 30.März 1993
- DATABASE WPI Section Ch, Week 8515 Derwent Publications Ltd., London, GB; Class D13, AN 85-089469 XP002056753 & JP 60 037 934 A (COSMOS SHOKUHIN KK) , 27.Februar 1985
- DATABASE WPI Section Ch, Week 9134 Derwent Publications Ltd., London, GB; Class A96, AN 91-248698 XP002056754 & JP 03 161 448 A (ASAHI CHEM IND CO LTD) , 11.Juli 1991
- DATABASE WPI Section Ch, Week 9202 Derwent Publications Ltd., London, GB; Class D13, AN 92-013085 XP002056755 & JP 03 263 499 A (ASAHI DENKA KOGYO KK) , 22.November 1991

## Beschreibung

Die vorliegende Erfindung betrifft neue, stabile, kaltwasserdispergierbare pulverförmige Präparate von einem mikrobiell hergestellten Oel, nachstehend SCO (**S**ingle **C**ell **O**il) genannt, welches reich an Arachidonsäure, nachstehend AA (**A**rachidonic **A**cid) genannt, ist, sowie ein Verfahren zu deren Herstellung.

Auf dem Gebiet der menschlichen Ernährung spielen kaltwasserdispergierbare Präparate von fettlöslichen Substanzen eine wichtige Rolle. In der Regel sind solche Präparate in Form von Emulsionen oder Trocken-pulvern im Handel, und zwar wegen der Wasserunlöslichkeit der fettlöslichen Wirkstoffe oder deren mehr oder weniger ausgeprägten Instabilität und schlechten Handhabbarkeit. Gemeinsam bei derartigen Präparaten sind die Wirkstoffe, d.h. die fettlöslichen Substanzen, in der Regel mittels einer Matrix-Komponente (Schutzkolloid) umhüllt. Diese Matrix-Komponente ist u.a. für den Schutz des Wirkstoffes bzw. für dessen Stabilisierung, für eine optimale Resorption und für die allfällig nötige Wasserdispergierbarkeit des Endpräparates verantwortlich.

Die europäische Patentanmeldung EP-A-0 347 751 beschreibt stabile, kaltwasserdispergierbare, flüssige oder pulverförmige Präparate fettlöslicher Substanzen, welche als Schutzkolloid Fischgelatine enthalten. Als fettlösliche Substanzen werden u.a. polyungesättigte Fettsäuren vorgeschlagen.

Ein Verfahren zur Gewinnung von Öl-in-Wasser Emulsionen bestehend aus bis zu 70 Gewichtsprozenten Fett und Öl, die polyungesättigte Fettsäuren enthalten, sowie aus einem Proteinanteil von bis zu 50 Gewichtsprozenten, welches zu Präparaten mit stabilen Geschmackseigenschaften und geringer Neigung zu oxydativem Verfall führt ist in der japanischen Patentanmeldung JP 05 078 692 A erwähnt.

Die deutsche Offenlegungsschrift DE 36 03 000 A beschreibt Fettmischungen für Säuglingsnahrung, die einen hohen Anteil an polyungesättigten Fettsäuren und Cholesterin enthält.

Erfindungsgemäss hat sich nun herausgestellt, dass sich ein besonders stabiles und gut handhabbares Präparat aus dem eingangs genannten SCO herstellen lässt, wenn man als Schutzkolloid Fischgelatine verwendet und ein Antioxydans beigibt. Als Antioxydantien werden verwendet:
a) für die Oelphase: Tocopherol, Ascorbinsäureester oder ein Gemisch aus Ascorbinsäureester und Tocopherol, wobei der Ascorbinsäureester vorzugsweise Ascorbylpalmitat ist;
b) für die wässrige Phase: Alkali- oder Erdalkalisalze der Ascorbinsäure, vorzugsweise Na-Ascorbat.

Eine besonders bevorzugte Fischgelatine ist die unter dem Namen "Norland HiPure Fish Gelatin" erhältliche Gelatine der Firma Norland Products Inc., 695 Joyce Kilmer Ave., New Brunswick, N.J., USA.

Zwar kann irgendein Tocopherol in der vorliegenden Erfindung verwendet werden, wobei Beispiele derartiger Tocopherole α-Tocopherol, γ-Tocopherol oder eine Mischung natürlicher Tocopherole sind, doch in einer bevorzugten Ausführungsform wird eine Mischung natürlicher Tocopherole verwendet.

Das Ascorbylpalmitat und Tocopherol werden vorzugsweise in einem Verhältnis von 1:5 bis 1:25, insbesondere 1:5, verwendet, und die Gesamtmenge, die dem SCO zugesetzt wird, beträgt mit Vorteil 1200 - 5250 Teile pro Million, insbesondere 1200 - 2000, vorzugsweise etwa 1200 Teile pro Million. Das Ascorbylpalmitat und Tocopherol kann als Mischung oder einzeln zugesetzt werden. Wird Tocopherol allein verwendet, so beträgt die Gesamtmenge mit Vorteil 1000 - 5000 Teile pro Million, insbesondere 1000 - 2000, vorzugsweise etwa 1000 Teile pro Million.

Die erfindungsgemässen Präparate können im Prinzip dadurch hergestellt werden, dass man eine wässrige Emulsion des SCO und der Fischgelatine zubereitet und diese Emulsion in ein Trockenpulver überführt. Dieses Herstellungsverfahren stellt einen weiteren Gegenstand der vorliegenden Erfindung dar.

In der Regel werden zunächst die Fischgelatine und gegebenenfalls Hilfsstoffe in Wasser gelöst, was zweckmässigerweise durch kräftiges Rühren beschleunigt wird. Der wässrigen Phase wird ein Antioxydans, beispielsweise Na-Ascorbat, zugefügt.

Uebliche Hilfsstoffe sind beispielsweise Mono- und Di-Saccharide; Zuckeralkohole; Stärkederivate, z.B. Maltodextrin; Milchproteine, z.B. Natriumkaseinat; oder auch pflanzliche Proteine, z.B. Sojaprotein, Kartoffelprotein und Weizenprotein. Zudem erweist es sich als vorteilhaft, das Lösen der Fischgelatine bei Raumtemperatur oder erhöhter Temperatur, insbesondere im Temperaturbereich von 20°C bis 90°C, vorzugsweise 50°C bis 70°C, durchzuführen. Auf diese Weise wird die sogenannte Matrix erhalten. Dann wird das durch ein Antioxydans stabilisierte SCO in diese Matrix hineinemulgiert, vorteilhafterweise durch Homogenisieren bei Atmosphärendruck oder erhöhtem Druck bis 1000 bar (100MPa), vorzugsweise bei 300-500 bar (30-50 MPa), oder auch mittels Ultraschall oder ähnlicher Techniken. Der Druck und die Temperatur sind bei diesem Vorgang keine kritischen Parameter, und das Ganze kann ohne weiteres bei Temperaturen von etwa Raumtemparatur bis etwa 70°C, insbesondere zwischen etwa 60°C und 70°C, und Atmosphärendruck durchgeführt werden.

Das Gewichtsverhältnis von SCO zu den im Endprodukt letztlich vorhandenen Begleitstoffen (Fischgelatine, Zucker usw.) beträgt in der Regel etwa 20:80 bis etwa 80:20, wobei die genauen Mengenverhältnisse abhängig sind vom jeweiligen biologischen Bedarf an AA und von der Forderung nach gleichmässiger und ausreichend feiner Verteilung der Endpräparate in den zur Konsumation vorgesehenen Anwendungsformen.

Die Ueberführung einer so hergestellten Emulsion, die je nach Bestandteilen im allgemeinen von ca. 20 bis ca. 80 Gewichtsprozent an SCO enthält, in Trockenpulver, kann z.B. durch Sprühtrocknung, das Doppeldispergier-Verfahren oder auch das Stärke-Catch-Verfahren erfolgen. Bei letzterem Verfahren werden die versprühten Emulsionströpfchen in einem Stärkebett aufgefangen und anschliessend der Trocknung zugeführt. Falls notwendig, kann die zu versprühende Emulsion mit Wasser verdünnt werden.

Im allgemeinen weisen die erfindungsgemässen Präparate eine gute Kaltwasser-Dispergierbarkeit sowie eine gute Fliessbarkeit auf.

Die erfindungsgemässen Präparate können für die menschliche Ernährung, insbesondere der von Neugeborenen, Verwendung finden.

Die vorliegende Erfindung wird durch die nachfolgenden Beispiele veranschaulicht.

### Beispiel

In einem 600 ml Becherglas wurden 44.2 g Fischgelatine getrocknet und 44.2 g Kristallzucker und 8.6 g Natriumascorbat vorgelegt. Dann wurden 80 ml entionisiertes Wasser zugesetzt, und das Ganze unter Rühren mit einer Minzerscheibe (1000 U/Min.) bei 50°C in Lösung gebracht. Hierauf wurden 60 g eines 50% AA enthaltenden SCO, welches mit einem Gemisch von 1000 ppm einer Mischung natürlicher Tocopherole und 200 ppm Ascorbylpalmitat stabilisiert wurde, in diese Matrix hineinemulgiert und 15 Minuten nachgerührt (während des Emulgierens und Nachrührens betrug die Tourenzahl der Minzerscheibe 4800 U/Min.). Nach dieser Zeit wies die innere Phase der Emulsion eine mittlere Teilchengrösse von etwa 180 nm auf. Die Emulsion wurde dann mit 90 ml entionisiertem Wasser verdünnt und auf 65°C erwärmt. In einer Laborsprühwanne wurden dann ca. 1 kg mittels Kieselsäure fluidisierte Stärke vorgelegt und auf ca. 5°C gekühlt. Hierein wurde dann die Emulsion mittels einer rotierenden Sprühdüse eingesprüht. Die so erhaltenen, mit Stärke umhüllten Partikel wurden dann von der überschüssigen Stärke abgesiebt und bei Raumtemparatur mittels Pressluft getrocknet. Man erhielt ca. 190 g Trockenpulver mit einem AA-Gehalt von 17%.

## Patentansprüche

1. Stabile, kaltwasser-dispergierbare pulverförmige Präparate von einem mikrobiell hergestellten Öl, welches reich an Arachidonsäure ist, **dadurch gekennzeichnet, dass** sie als Matrix-Komponente Fischgelatine und Tocopherol, Ascorbinsäureester oder ein Gemisch von Ascorbinsäureester und Tocopherol als Antioxydans für die ölige Phase enthalten und als Antioxydans für die wässrige Phase Alkali- oder Erdalkalisalze der Ascorbinsäure enthalten.

2. Präparat nach Anspruch 1, **dadurch gekennzeichnet, dass** es als Antioxydans für die ölige Phase Tocopherol oder ein Gemisch aus Ascorbylpalmitat und Tocopherol enthält.

3. Präparat nach Anspruch 1 oder 2, **dadurch gekennzeichnet, dass** es Ascorbylpalmitat und Tocopherol in einem Verhältnis von 1:5 bis 1:25, insbesondere 1:5, enthält.

4. Präparat nach einem der Ansprüche 1-3, **dadurch gekennzeichnet, dass** die Gesamtmenge an Ascorbylpalmitat und Tocopherol 1200 - 5250 Teile pro Million beträgt.

5. Präparat nach Anspruch 4, **dadurch gekennzeichnet, dass** die Gesamtmenge an Ascorbylpalmitat und Tocopherol 1200 - 2000 Teile pro Million, insbesondere 1200 Teile pro Million, beträgt.

6. Präparat nach einem der Ansprüche 1 bis 5, **dadurch gekennzeichnet, dass** das Tocopherol eine Mischung natürlicher Tocopherole ist.

7. Präparat nach einem der Ansprüche 1 bis 6, **dadurch gekennzeichnet, dass** es als Antioxydans für die wässrige Phase Natriumascorbat und gegebenenfalls einen Hilfsstoff enthält.

8. Verfahren zur Herstellung von Präparaten gemäss Anspruch 1, **dadurch gekennzeichnet, dass** man
a) Fischgelatine und Alkali- oder Erdalkalisalze der Ascorbinsäure und gegebenenfalls einen Hilfsstoff in Wasser löst,
b) das durch Tocopherol, Ascorbinsäureester ein Gemisch aus Ascorbinsäureester und Tocopherol stabilisierte mikrobiell hergestellte Öl in die wässrige Matrix hinein emulgiert und
c) die so hergestellte Emulsion in ein Trockenpulver überführt.

## Claims

1. Stable, cold water-dispersible pulverous preparations of a microbially produced oil, which is rich in arachidonic acid, said preparations containing fish gelatine as the matrix component and tocopherol, an ascorbic acid ester or a mixture of an ascorbic acid ester and tocopherol as antioxidant for the oily phase and as antioxidant for the aqueous phase alkali or alkaline earth salts of ascorbic acid.

2. A preparation according to claim 1, which contains tocopherol or a mixture of ascorbyl palmitate and tocopherol as antioxidant for the oily phase.

3. A preparation according to claim 1 or 2, which contains ascorbyl palmitate and tocopherol in a ratio of 1:5 to 1:25, especially 1:5.

4. A preparation according to any one of claims 1-3, wherein the total amount of ascorbyl palmitate and tocopherol is 1200-5250 parts per million.

5. A preparation according to claim 4, wherein the total amount of ascorbyl palmitate and tocopherol is 1200-2000 parts per million, especially 1200 parts per million.

6. A preparation according to any one of claims 1 to 5, wherein the tocopherol is a mixture of natural tocopherols.

7. A preparation according to any one of claims 1 to 6, which contains as antioxidant for the aqueous phase sodium ascorbate and if necessary an adjuvant.

8. A process for the manufacture of preparations, according to claim 1, which process comprises:
a) dissolving fish gelatine and alkali or alkaline earth salts of ascorbic acid, and if necessary an adjuvant in water,
b) emulsifying the microbially produced oil stabilised by tocopherol, an ascorbic acid ester or a mixture of an ascorbic acid ester and tocopherol in the aqueous matrix and
c) converting the thus-produced emulsion into a dry powder.

## Revendications

1. Préparations en poudre, stables, dispersibles dans l'eau froide, d'une huile préparée par voie microbienne, qui est riche en acide arachidonique, **caractérisées en ce qu'**elles contiennent en tant que composant de matrice de la gélatine de poisson et du tocophérol, un ester de l'acide ascorbique ou un mélange d'un ester de l'acide ascorbique et de tocophérol en tant qu'antioxydant pour la phase huileuse, et, en tant qu'antioxydant pour la phase aqueuse, contiennent des sels de métaux alcalins ou alcalino-terreux de l'acide ascorbique.

2. Préparation selon la revendication 1, **caractérisée en ce qu'**elle contient en tant qu'antioxydant pour la phase huileuse du tocophérol ou un mélange de palmitate d'ascorbyle et de tocophérol.

3. Préparation selon la revendication 1, **caractérisée en ce qu'**elle contient du palmitate d'ascorbyle et du tocophérol selon un rapport de 1:5 à 1:25 et en particulier de 1:5.

4. Préparation selon l'une des revendications 1 à 3, **caractérisée en ce que** la quantité totale du palmitate d'ascorbyle et du tocophérol est de 1200 à 5250 parties par million.

5. Préparation selon la revendication 4, **caractérisée en ce que** la quantité totale du palmitate d'ascorbyle et du tocophérol est de 1200 à 2000 parties par million, en particulier de 1200 parties par million.

6. Préparation selon l'une des revendications 1 à 5, **caractérisée en ce que** le tocophérol est un mélange de tocophérols naturels.

7. Préparation selon l'une des revendications 1 à 6, **caractérisée en ce qu'**elle contient en tant qu'antioxydant pour la phase aqueuse de l'ascorbate de sodium et éventuellement un adjuvant.

8. Procédé de production de préparations selon la revendication 1, **caractérisée en ce que**
a) on dissout dans de l'eau de la gélatine de poisson et des sels de métaux alcalins ou alcalino-terreux de l'acide ascorbique et éventuellement un adjuvant,
b) on introduit par émulsification dans la matrice aqueuse l'huile préparée par voie microbienne et stabilisée par du tocophérol, un ester de l'acide ascorbique ou un mélange d'un ester de l'acide ascorbique et de tocophérol, et
c) on convertit en une poudre sèche l'émulsion ainsi préparée.
